# EUROPEAN PATENT SPECIFICATION

(11) **EP 2 459 269 B1**
(45) Date of publication and mention of the grant of the patent: **01.05.2019**
(21) Application number: 10804938.8
(22) Date of filing: 26.07.2010
(51) Int. Cl.: A61M 37/00, A61M 1/16

(54) **MODULAR HEMOFILTRATION APPARATUS FOR CARRYING OUT NEONATAL AND PEDIATRIC CRRT**
MODULARE HÄMOFILTRATIONSVORRICHTUNG ZUR CRRT-DURCHFÜHRUNG BEI NEUGEBORENEN UND KINDERN
APPAREIL MODULAIRE D'HÉMOFILTRATION POUR L'EXÉCUTION D'UNE CRRT NÉONATALE OU PÉDIATRIQUE

(30) Priority: 27.07.2009 US 228870 P
(43) Date of publication of application: 06.06.2012
(73) Proprietor: B. Braun Avitum AG, 34212 Melsungen (DE)
(72) Inventor: DELMAGE, Michael, Napa CA 94558 (US); PETERS, Harold, Snow Hill NC 28580 (US); COOPER, Tommy, Friendswood TX 77546 (US); MACHA, Steve, Sugar Land TX 77478 (US)
(74) Representative: Winter, Brandl, Fürniss, Hübner, Röss, Kaiser, Polte - Partnerschaft mbB
(86) International application number: PCT/US2010/043266
(87) International publication number: WO 2011/014463

(56) References cited:
- WO-A1-00/66197
- WO-A2-01/45769
- US-A1- 2004 267 184
- US-A1- 2006 184 084
- US-A1- 2007 066 928
- US-A1- 2007 138 069
- US-A1- 2007 185 429
- US-A1- 2008 045 877
- US-A1- 2008 154 170
- US-A1- 2009 084 717
- US-A1- 2009 084 717

## Description

### BACKGROUND OF THE INVENTION

Hemodialysis systems have been designed to carry out blood therapy procedures such as slow continuous ultrafiltration (SCUF), continuous veno-venous hemofiltration (CWH), continuous veno-venous hemodialysis (CWHD) or continuous veno-venous hemodiafiltration (CWHDF). These continuous renal replacement therapies, referred to as CRRT, are designed for removal of metabolic waste and excess fluid from patients in fluid overload and who need renal support. Presently available extracorporeal blood treatment apparatus often requires inconvenient and time consuming setup procedures including cleaning and/or replacing the blood and/or fluid tubing for different patients and for different therapies. Such procedures may require the apparatus to be removed from a patient's bedside or room to another location, or replacing an apparatus with a system that is set up and configured for carrying out a specific therapy.

U.S. Pat. No. 5,910,252 describes an apparatus configured for performing the different blood therapies and provides means for selecting one of the therapies to be carried out. The described apparatus is an assembly of all pumps, tubing, multiple fluid supply reservoirs, waste fluid container and filter cartridge necessary for performing any one of the selected blood therapies.

U.S. Pat. No. 6,200,485 describes another multipurpose hemofiltration system comprising an assembly of a blood filter cartridge, pumps, fluid reservoir and waste fluid container, components for comparing the weights of the fluid reservoir and waste fluid container and means for controlling the pump operations and rate in response to the compared weights during the therapy.

U.S. patent application US 2009/084717 A1 describes a modular hemofiltration apparatus with removable panels for multiple and alternate blood therapy. An apparatus for performing blood therapy has a plurality of pumps for engaging blood and fluid tubing and is characterized by a plurality of manually mounted and disengagable panels installed on the sides of the apparatus housing, the panels having pump engaging tubing mounted on the inside of the respective panels.

U.S. patent application US 2008/154170 A1 describes a total fluid loss control system. In the application a system or method controls total fluid loss (TFL) in a patient undergoing hcmofiltration therapy administered through an extracorporeal circuit by frequently calculating and storing a retrievable value representing TFL. At session start or restart, the value is read to determine whether TFL is out of tolerance, and if so, fluid balance is restored before administering a prescribed therapy. The TFL value is calculated during therapy by measuring fluid added to the circuit and fluid removed from the circuit, and the result is stored as an updated value. TFL drifts out of tolerance fluid flow rates may be temporarily changed until a desired fluid balance is restored. The system may include a microprocessor based advanced controller receiving the fluid measurements as feedback for regulating substitution fluid and filtrate flow rates, and updating the TFL value in non-volatile memory.

The international patent application WO 2001/45769 describes systems and methods for controlling blood flow and waste fluid removal during hemofiltration. During hemofiltration, blood is conveyed from an individual through a blood line into a hemofilter to remove waste fluid. Waste fluid is conveyed from the hemofilter through a waste removal line at a waste removal rate. Waste fluid pressure is sensed in the waste removal line. A controller adjusts the waste removal rate to maintain waste fluid pressure at a predetermined set value.

A Prismaflex™ system marketed by Gambro of Lakewood, Colo. offers selection of different CRRT therapies. The system allows the user to select a prepackaged, preassembled assembly incorporating all of the components including specific column and type of filter membrane or membrane filter surface area and all preconnected tubing for carrying out the selected therapy.

In U.S. patent application Ser. No. 12/183,537, filed Jul. 31, 2008 (TRANSVI.024A), there is described a modular hemofilter apparatus having removable panels for multiple and alternate blood therapy. The apparatus and system described in the aforesaid application provides a flexible treatment system characterized by a panel assembly having removable and disposable panels installed on the apparatus housing control unit whereby filter columns and/or tubing sets mounted on the panels may be replaced with filters and/or panels having different tubing configurations to accommodate different blood treatment therapies.

In U.S. patent application Ser. No. 12/577,578 filed Oct. 12, 2009 (TRANSVI.025A), the aforesaid modular hemofiltration apparatus with removable panels is further described including special and unique panel designs and tubing configurations. In U.S. patent application Ser. No. 12/577,513 filed Oct. 12, 2009 (TRANSVI.026A), there are disclosed components and features for readily and efficiently manually mounting and removing the panels and filter cartridges by an operator. In U.S. patent application Ser. No. 12/608,806 filed Oct. 29, 2009 (TRANVSVI.028A), there is described a modular hemofiltration apparatus with interactive operator instructions and control system characterized by operator inputs for selecting CRRT patient therapy, changing panel sets, replacing a filter cartridge and changing to a different patient therapy from a currently running patient therapy.

### SUMMARY OF THE INVENTION

The invention is defined by the features of independent claim 1.

The apparatus described herein provide a unique safety paradigm for CRRT therapies. This system is designed to ensure that warnings and alarms are keyed to the maximum fluid gain or loss calculation in such a way as to prevent volumetric errors beyond 10% of a patient's blood volume at all times. The system provides operator input for setting a patient weight from zero kg up to about 20 kg, blood pump flow rate and fluid pump flow rates, and calculates and displays maximum allowable fluid gain or loss based solely on the patient weight settings and triggers alarm and/or stops pump operation or termination of CRRT in response to patient fluid gain or loss in excess of the maximum. In some embodiments, the control system provides one or more warnings and/or alarms at predetermined fluid gain or loss levels below the calculated or selected maximum allowable. In other embodiments, the control system will stop pump operation after a certain number of warnings and/or alarms. In some embodiments, the control system provides for maximum fluid gain or loss over selected or predetermined time periods. In one embodiment, the control system provides for the maximum fluid gain or loss during a time period of about 3 hours. In one embodiment, the apparatus includes an interactive operator control system with inputs for operator setting replacement fluid rate, dialysate rate and net patient fluid removal rate with the controller automatically adjusting effluent removal rate to maintain operator set net fluid removal rate. In another embodiment, the interactive operator control system provides operator input for setting an excess fluid gain or loss limit below the calculated maximum. In another embodiment, the controller is configured to automatically trigger stopping pump operation or CRRT termination in response to fluid gain or loss in excess of the maximum setting. In yet another embodiment, the controller is configured to prevent operator input of flow rates in excess of a maximum input flow rate displayed on the interface screen. In another embodiment, the controller is configured to calculate and set the maximum allowable fluid gain or loss at 7 ml/kg for patient weight settings from zero kg to 20 kg. In another embodiment, the controller sets maximum allowable fluid gain or loss of 42 ml for patients weighing between zero kg to about 6 kg and maximum allowable fluid gain or loss of 7 ml/kg for patient weights from 6 kg to 20 kg. In another embodiment, the controller is configured to automatically adjust effluent removal rate to maintain operator set net fluid removal rate without adjusting operator set dialysate and replacement fluid flow rates. In another embodiment, the apparatus controller is configured to automatically set a zero net fluid removal in response to operator input of patient weight of between about zero kg and about 20 kg.

The aforesaid embodiments for carrying out neonatal and pediatric CRRT utilize an apparatus described herein which comprises a control unit with blood and fluid pumps, manually installed and replaceable panel kits mounted on the control unit having blood and fluid supply tubing on the panels, a replaceable filter cartridge, a controller CPU configured for operating the system including blood pump and fluid pumps and an interactive operator control system with an operator interface screen operatively connected to the controller. The controller CPU comprises one or more microprocessors provided with software configured to operate the apparatus in response to operator input selections and provide apparatus operating instructions and status of selected therapy parameters. The interactive operator control system is characterized by operator inputs for selecting a CRRT patient therapy, changing the panel sets, replacing the filter cartridge and changing to a different patient therapy from a currently running patient therapy. The operator input control panel also provides step-by-step operator instructions for changing the panel kit, replacing a filter cartridge and changing patient therapy during a running patient therapy. The interactive user control system utilizes an operator interface touch-screen with graphic controls whereby the operator may select system operations and is provided with instructions for carrying out the selected system operations and patient therapy sessions. The system also provides operator selection of temporary patient disconnect and later start procedure during a current selected therapy session as well as detailed operator instructions for carrying out the procedures. These as well as other components, features, parameters and advantages of the apparatus and its use will be further evident from the following detailed description.

### BRIEF DESCRIPTION OF THE DRAWINGS

FIGS. 1 and 2 are perspective views of the hemofiltration control apparatus showing the front and different sides of the panel assembly mounted on the control unit housing;
FIG. 3 is a schematic view of the modular blood therapy apparatus showing the interior panel fluid and blood tubing layout design with three fluid holding bags in fluid communication with the tubing;
FIG. 4 shows the interior panel surfaces with mounted tubing on blood supply and fluid tubing panels and a connected center panel and mounted hemofilter cartridge;
FIG. 5 shows an operator interface screen for inputting the patient weight; and
FIG. 6 shows the operator interface flow rate setup screen.

### DETAILED DESCRIPTION OF THE EMBODIMENTS

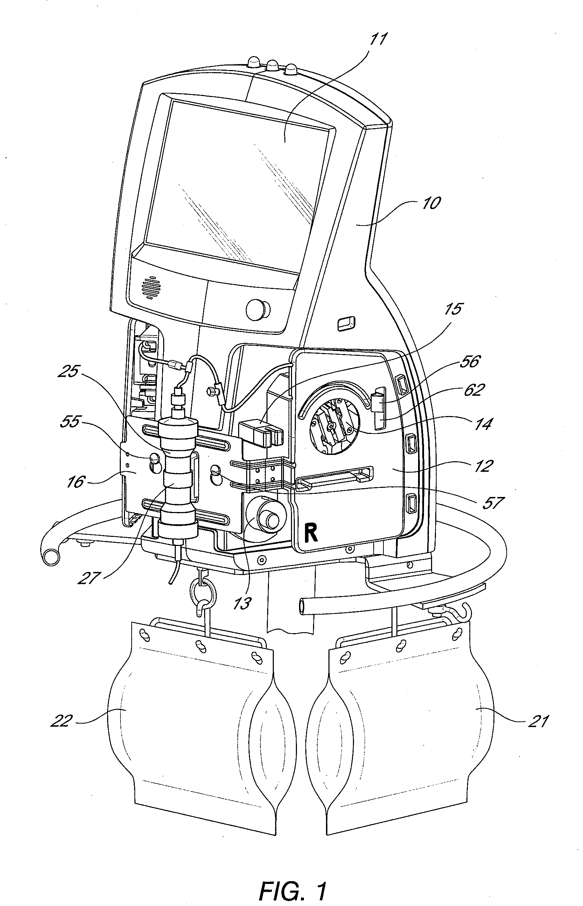

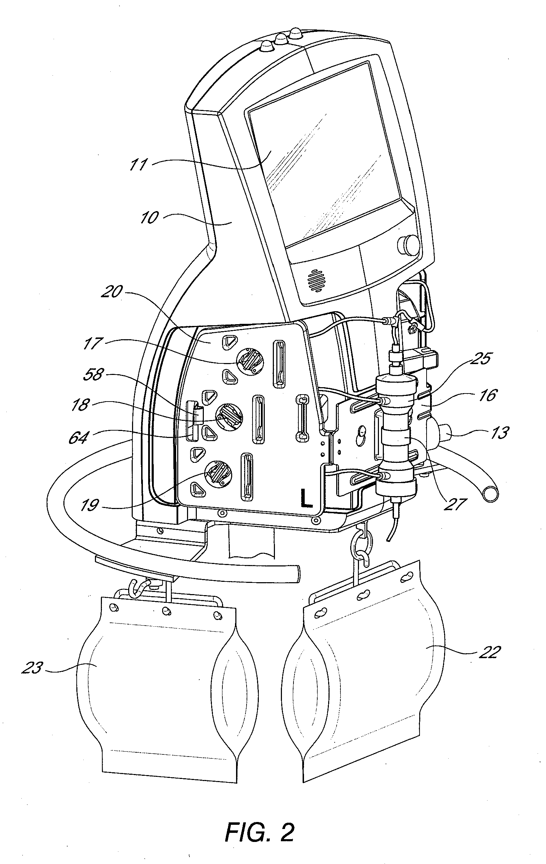

FIGS. 1 and 2 show a hemofiltration assembly including a control unit housing 10 with an operator interface touch-screen 11, illustrating opposite side views from the front corners of the apparatus. In FIG. 1, the blood side is shown with blood pump rotor 14 viewable through an opening in blood panel 12. In FIG. 2, three fluid pump rotors 17, 18, 19 are visible through viewing ports in fluid panel 20. On the front, a filter cartridge panel 16 with mounted hemofilter 25 is shown. The hemofilter, which is removable, is secured on the front panel with a filter cartridge strap 27 threaded through slots in the front panel. The strap may be conveniently provided with contact-type, adjustable securing components such as hook and loop (Velcro®) components which readily provide for securing different sized filter cartridges. Alternatively, the contact surfaces of the hemofilter and front panel may be provided with contact attaching means. The panels of the panel assembly set are mounted on the control unit housing by features and components as will be explained further hereinafter. In FIG. 1, an air detector 15 and return line clamp 13 are shown, and in FIGS. 1 and 2 fluid holding bags 21, 22, 23 are also shown.

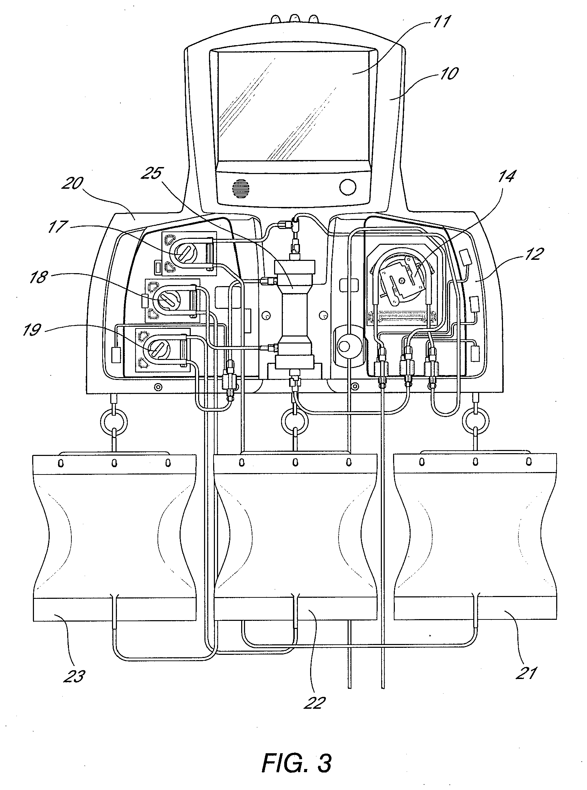

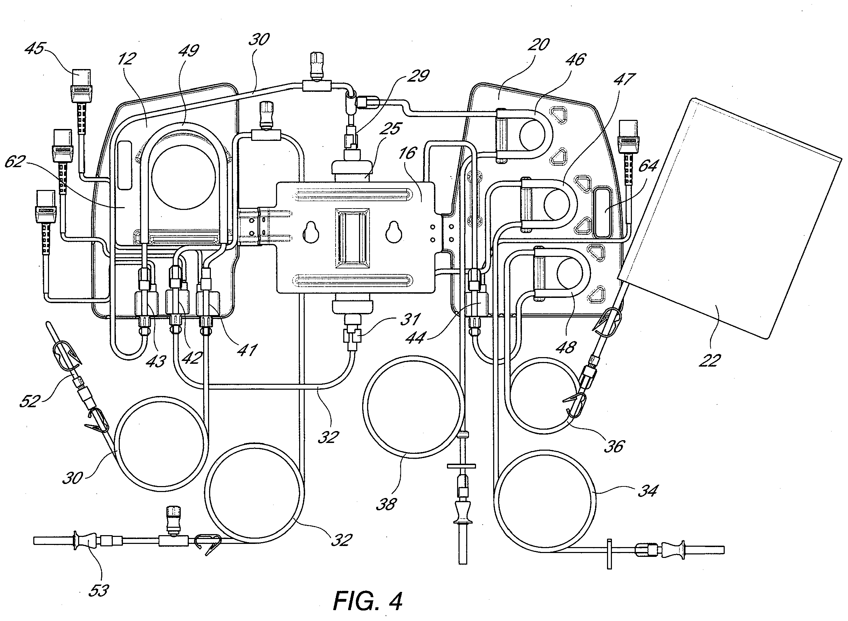

In FIGS. 3 and 4 there is shown the interior of the three-panel set with a tubing mounted on or adjacent to the interior panel surfaces and the relationship of the tubing as it is positioned for engagement with the respective blood pump and fluid pumps. Connections between sections or segments of the tubing with one another, with the filter cartridge, fluid pressure transducers, fluid supply containers and an effluent bag as well as to cannulae or catheters for directing blood to or from a patient are also shown.

The blood panel 12 includes blood supply tubing mounted along the interior, generally flat surface of the panel. An arched, upwardly slanted U-shape bend 49 of the blood supply tubing engages the rotors of blood pump 14 when the blood panel is securely mounted on the control unit housing. On the blood panel are positioned three pressure transducers 41, 42, 43 through which different sections of the blood supply tubing channels pass. The end of blood inlet line 30 is attached to a patient access device such as a needle cannula or catheter assembly 32 and includes a clamp for closing off the blood supply tubing. A blood pump 14 (FIGS. 1, 3) pumps blood along blood supply tubing blood inlet line 30 from the patient to the filter cartridge 25 via a blood inlet adapter 29. A blood return line 32 secured to an opposite end of the filter cartridge via a blood return adapter 31 includes segments directing the return line through the pressure transducer 42 and to a needle cannula assembly and/or catheter 53. Each of the pressure transducers has a pressure signal cable attached to a transducer plug 45, to be inserted in a socket on the control unit housing and for monitoring pressure signals from the respective pressure transducers along the blood inlet and return line segments.

The fluid side of the assembly comprises fluid panel 20 with tubing mounted along the generally flat interior surface. The fluid tubing is configured and shaped to engage three fluid pumps 17, 18, 19 secured to the control unit. Similar to the configuration of the blood supply tubing, three arched or U-shaped tubing segments 46, 47, 48 are provided to engage the rotors of the respective fluid pumps for driving fluids through the tubing. The different tubing segments and cooperating fluid pumps direct dialysate fluid, replacement fluid, saline/anticoagulant fluid, depending on the apparatus and blood treatment configuration, and effluent or waste fluid from the filter to the effluent container or bag. More specifically, replacement fluid line 38 directs replacement fluid from replacement fluid bag 21 via arched tubing section 46 to blood inlet line 30 and into the upper end of the filter cartridge 25. A second fluid tubing line directs dialysate fluid from dialysate fluid bag 23 into the side of the filter cartridge 25 via arched tubing section 47. A third fluid tubing section directs waste effluent from the filter cartridge to effluent fluid collection bag 22 via arched tubing section 48. A pressure transducer 44 is also positioned on the fluid panel. In a preferred embodiment, all of the pressure transducers are positioned on the respective panels such that when the panels are securely mounted on the control unit housing, the pressure transducers are at the same vertical elevation so that accurate pressure readings can be taken and compared, without further adjustment, which would otherwise be required to compensate for differences in transducer elevations. More specific and detailed descriptions of the tubing layouts on the respective blood and fluid panels are described and shown in aforesaid U.S. patent application Ser. No. 12/183,527 (TRANSVI.024A) and U.S. patent application Ser. No. 12/577,578 (TRANSVI.025A).

The above-described apparatus is configured for carrying out selected CRRT blood therapy procedures including SCUF, CVVH, CWHT and CWHDF. Such procedures are well known in the art and further described in detail in the aforesaid applications, particularly TRANSVI.028A. As previously described, the apparatus including the interactive operator system and interface screen operatively connected to the controller provide for selecting and carrying out the different therapies, as well as for terminating a currently running therapy and selecting an alternate therapy. As also previously described, the operator interface screen provides operator instructions for carrying out the therapies, including setting up the apparatus for selectively changing or installing the fluid bags during setup and running patient therapy, replacing panel kits and/or a filter cartridge during a running patient therapy session, and the like.

In carrying out pediatric and neonatal CRRT operating the apparatus at diminished blood and fluid flows from those typically used for adult therapy assists in preventing excessive fluid removal (effluent) and gives an operator substantially better control of net fluid removal. For example, for most adult CRRT therapy, a net removal setting of 0-2,000 ml/hr and a 3-hour excess fluid gain or loss limit of 125-400 ml is typically allowable on a flow rate setup by the operator. However, for neonatal and pediatric patients up to 20 kg range, such high net removal and excess fluid gain or loss limits are excessive.

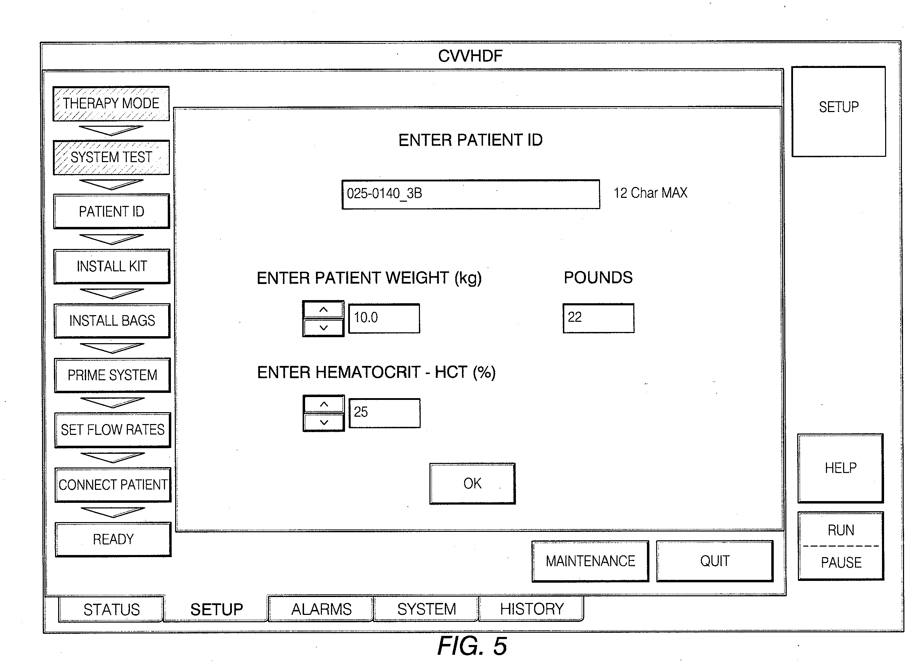

In the apparatus described herein, in one embodiment, the interactive operator control system provides operator inputs for setting a patient weight at between zero kg and about 20 kg. Such an operator interface screen is illustrated in FIG. 5. To enter the neonatal or pediatric patient weight, the operator touches the "setup" tab on the patient ID screen and enters the patient weight. In response to such input, the controller is configured to calculate and display a maximum allowable fluid gain or loss based on the patient weight settings. However, the apparatus need not be limited to neonatal and pediatric patient use, and preferably allows for input of adult patient weight settings and calculates maximum allowable fluid gain or loss, warnings, alarms, pump stop and CRRT termination as well.

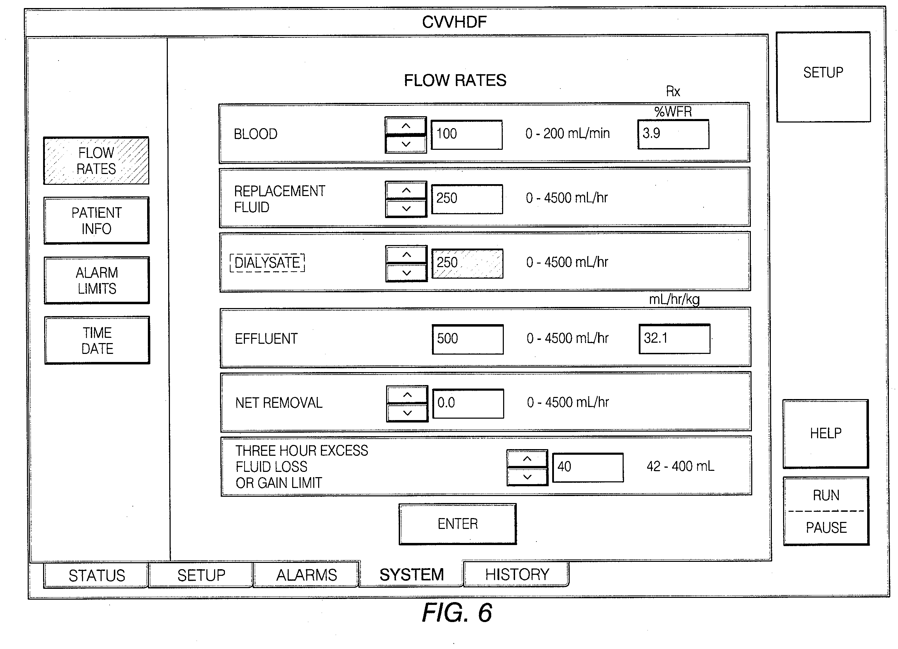

In FIG. 6, the flow rate setup screen is also shown, which screen appears when the operator presses the "system" tab. With the neonatal or pediatric patient weight set between zero kg and about 20 kg (FIG. 5), the controller is configured to calculate and display on the flow rate setup screen the maximum allowable fluid gain or loss based solely on the patient weight settings. Moreover, the controller is configured to trigger an alarm and/or stop operation of all pumps or termination of a CRRT session in response to patient fluid gain or loss in excess of the maximum allowable.

In some embodiments, the controller is configured to calculate, set and trigger one or more warnings and/or alarms for patient fluid gains or losses below the maximum allowable, thereby alerting an operator. For example, the controller may be configured to trigger a warning when a first fluid gain or loss is exceeded and trigger an alarm when a second, greater volume of fluid is gained or lost. Such warnings and alarms may also be set for fluid gains or losses over selected time periods. The controller may also be configured to stop the pump and terminate CRRT after a number of warnings and/or alarms.

In one embodiment, the controller is configured to trigger a warning or alarm as the 3-hour fluid gain or loss approaches 7 ml/kg of patient body weight for patient weight of 20 kg or less. In another embodiment, the controller is configured to trigger a warning or alarm and/or shutting down pump operation of the 3-hour fluid gain or loss in excess of 42 ml for patient weight settings of 6 kg or less. In yet another embodiment, the controller is configured to trigger a warning or alarm and/or shut down pump operation of 3-hour fluid gain or loss in excess of 140 ml for any patient weight settings of 20 kg or more.

In FIG. 6, the maximum allowable fluid gain or loss is shown as a 3-hour excess fluid loss or gain limit. However, in other embodiments, the apparatus may be configured for a different maximum fluid gain or loss time period and the flow rate screens modified accordingly.

In another embodiment, the controller is configured to trigger a warning in response to patient net fluid gain or loss in excess of about 20 ml and trigger an alarm in response to patient net fluid gain or loss in excess of about 30 ml at patient weight settings of between zero kg and about 10 kg. In another embodiment, the controller is configured to trigger a warning in response to patient net fluid gain or loss in excess of about 35 ml and trigger an alarm in response to patient net fluid gain or loss in excess of about 50 ml at patient weight settings of between about 10 kg and about 15 kg. In another embodiment, the controller is configured to trigger a warning in response to patient net fluid gain or loss in excess of about 50 ml and trigger an alarm in response to patient net fluid gain or loss in excess of about 80 ml at patient weight settings of between about 15 kg and about 20 kg. In yet another embodiment, the controller is configured to trigger a warning in response to patient net fluid gain or loss in excess of about 60 ml and an alarm at fluid gain or loss in excess of about 100 ml at patient weight settings above about 20 kg.

In one embodiment, the controller provides for a maximum allowable fluid gain or loss of between about 30 ml and about 40 ml at patient weight settings of between zero kg and about 10 kg, between about 50 ml and about 70 ml at patient weight settings of between about 10 kg and about 15 kg, and between about 80 ml and about 120 ml at patient weight settings of between about 15 kg and about 20 kg. Again, the controller is configured to calculate and display such allowable maximum fluid gain or loss. In another embodiment, the interactive operator control system prevents operator input for setting a fluid gain or loss in excess of the computer provided allowable maximum. In yet another embodiment, the operator control system provides for operator input for setting such 3-hour excess fluid gain or loss limit below the computer provided allowable maximum. Again, as previously described, the 3-hour time limit embodiment shown may be changed to meet other standards or preferred time periods.

In another embodiment, the controller is configured to terminate a CRRT session in response to operator restarting pumps multiple times. For example, in an embodiment, where the controller stops pump operation in response to net fluid gain or loss in excess of the maximum allowable calculated for a patient weight setting, the operator may restart the pumps to continue therapy. However, the controller may be configured to allow such restarts only a limited number of times, e.g., 3-10 times, after which it will not allow any further restart and/or will initiate termination procedures for the CRRT session.

As illustrated in FIG. 6, the interactive operator control system input provides for setting replacement fluid rate, dialysate rate and net removal rate. In one embodiment, the controller is configured to automatically adjust the effluent removal rate to maintain the operator set net fluid removal rate based on the formula:
net removal=effluent removed-dialysate added-replacement fluid added,
without adjusting operator set dialysate and replacement fluid flow rates. In response to these operator inputs, the controller sets the appropriate fluid pump flow for carrying out the CRRT. In one embodiment, the controller is also configured to calculate and display on the interface screen maximum operator input flow rates for blood, dialysate, replacement fluid and net fluid removal in response to operator patient weight input settings of between about 5 kg and about 20 kg. Moreover, in another embodiment, the controller is configured to prevent operator input of any flow rate in excess of aforesaid maximum input flow rates displayed. In another embodiment, the maximum flow rates for patients weighing zero kg to 20 kg are:
blood-100 ml/minute,
dialysate fluid-250 ml/hr, and
replacement fluid-250 ml/hr.

Such a controller configuration still allows ample latitude for operator controlling desired blood and/or flow rates but prevents an operator from inadvertently or unknowingly prompting the apparatus to carry out neonatal or pediatric CRRT sessions at excessive flow rates as well as alarming an operator and shutting down the therapy session in response to excessive fluid gain or loss limits, the latter which may be caused by pump or other apparatus malfunction or failure.

In yet another embodiment, the controller is configured to set the net removal at 0 or up to a maximum of 2000 ml/hr. In typical adult CRRT sessions, net removal is set at, for example, between about 100 ml and about 500 ml or even up to 1,000 ml/hr. However, for pediatric and neonatal CRRT, such net removals are excessive, in view of the small range of error allowable for such patients with relatively small circulatory volumes. Similarly, the reduced replacement fluid and dialysate flow rates, as well as blood flow, gives the operator or physician flexibility in changing such flow rate within the parameters of the aforesaid maximum settings.

It is to be understood that the aforesaid flow rates have been described regarding CVVHD therapy, as shown in FIG. 6 and described further herein. However, the apparatus is not limited to a CVVHDF setup and may be applied to any of the aforesaid CRRT therapies for which the apparatus is capable of carrying out.

The computer is also configured to ensure that the panel kit installed is appropriate for the selected treatment. As well, the computer is configured to ensure that the appropriate filter has been installed. This is accomplished by a barcode scanner feature of the control unit for scanning barcodes on the panel kit and filter. The hemofilter used for neonatal and pediatric CRRT is smaller than that required for adult patients. Examples of useful hemofilters are Minntech HF 400, HF 700 or HF 1200, or other equivalent filters.

## Claims

1. An apparatus configured for carrying out Continuous Renal Replacement Therapies (CRRT) on patients weighing less than about 20 kg, comprising:
a control unit comprising:
a blood pump (14) and a plurality of fluid pumps (17, 18, 19) mounted thereon;
a controller configured for operating said blood pump (14) and said fluid pumps (17, 18, 19); and
an interactive operator control system comprising an operator interface screen operatively connected to said controller, wherein said controller comprises software configured to operate said apparatus in response to operator input selections; and
a replaceable panel kit configured to be manually mounted on and removed from said control unit comprising
a blood panel (12);
a fluid panel (20) comprising blood and fluid supply tubing, respectively, secured thereon and in operating engagement with said blood pump (14) and said fluid pumps (17, 18, 19), respectively;
a filter cartridge panel (16) secured to said blood panel (12) and said fluid panel (20); and
a replaceable filter cartridge (25) mounted thereon,
**characterized in that**,
said interactive operator control system includes operator inputs for setting patient weight, blood pump flow rate and fluid pump flow rates; and
said controller is configured to calculate and display maximum allowable fluid gain or loss based solely on patient weight settings between zero kg and about 20 kg and trigger an alarm and/or stop operation of said pumps (14, 17, 18, 19) or termination of CRRT in response to patient fluid gain or loss in excess of said maximum allowed fluid gain or loss.

2. An apparatus of claim 1 wherein said controller provides for maximum allowable fluid gain or loss of about 7 ml/kg for patient weight settings between zero kg and about 20 kg.

3. An apparatus of claim 1 wherein said controller provides for maximum allowable fluid gain or loss of about 42 ml for patient weight settings between zero kg and about 6 kg.

4. An apparatus of claim 3 wherein said controller provides for maximum allowable fluid gain or loss of about 7 ml/kg for patient weight settings between about 6 kg and about 20 kg.

5. An apparatus of claim 1 wherein the controller is configured to set a first and a second net fluid gain/loss and trigger a warning signal when patient fluid gain or loss is in excess of said first net fluid gain/loss and trigger an alarm when fluid gain or loss is in excess of said second net fluid gain/loss.

6. An apparatus of claim 1 wherein said fluid pumps (17, 18, 19) comprise a dialysate fluid pump, a replacement fluid pump and an effluent pump, wherein said operator inputs comprise replacement fluid rate, dialysate rate and net fluid removal rate and wherein said controller automatically adjusts effluent removal rate without adjusting operator set dialysate flow rate and replacement fluid flow rate to maintain operator set net fluid removal based on the formula:
net removal=effluent removed-dialysate added-replacement fluid added.

7. An apparatus of claim 1 wherein said controller is configured to trigger a warning in response to net fluid gain or loss in excess of about 20 ml and trigger said alarm in response to net fluid gain or loss in excess of about 30 ml at patient weight settings of between zero kg and about 10 kg.

8. An apparatus of claim 1 wherein said controller is configured to trigger a warning in response to net fluid gain or loss in excess of about 35 ml and trigger said alarm in response to net fluid gain or loss in excess of about 50 ml at patient weight settings of between about 10 kg and about 15 kg.

9. An apparatus of claim 1 wherein said controller is configured to trigger a warning in response to net fluid gain or loss in excess of about 50 ml and trigger said alarm in response to net fluid gain or loss in excess of about 80 ml at patient weight settings of between about 15 kg and about 20 kg.

10. An apparatus of claim 1 wherein the controller provides for maximum allowable fluid gain or loss of between about 30 ml and about 40 ml at patient weight settings of between zero kg and about 10 kg.

11. An apparatus of claim 1 wherein the controller provides for maximum allowable fluid gain or loss of between about 50 ml and about 70 ml at patient weight settings of between about 10 kg and about 15 kg.

12. An apparatus of claim 1 wherein the controller provides for maximum allowable fluid gain or loss of between about 80 ml and about 120 ml at patient weight settings of between about 15 kg and about 20 kg.

13. An apparatus of claim 1 wherein said interactive operator control system includes operator input for setting an excess fluid gain or loss limit below said allowable maximum.

14. An apparatus of claim 13 wherein said controller is configured to automatically trigger said CRRT termination in response to set fluid gain or loss in excess of said setting during a time period of up to about 3 hours.

15. An apparatus of claim 1 wherein said controller is configured to prevent operator input for setting a fluid gain or loss in excess of said maximum.

## Patentansprüche

1. Vorrichtung, die zum Durchführen von kontinuierlichen Nierenersatztherapien (CRRT) bei Patienten mit einem Gewicht von weniger als etwa 20 kg konfiguriert ist, aufweisend:
eine Steuereinheit, aufweisend:
eine Blutpumpe (14) und eine Vielzahl von Fluidpumpen (17, 18, 19), die darauf montiert sind;
eine Steuerung, die zum Betreiben der Blutpumpe (14) und der Fluidpumpen (17, 18, 19) konfiguriert ist; und
ein interaktives Bediensystem, das einen Bedienerschnittstellenbildschirm aufweist, der funktionsfähig mit der Steuerung verbunden ist, wobei die Steuerung eine Software aufweist, die konfiguriert ist, um die Vorrichtung in Reaktion auf die Auswahl von Bedienereingaben zu bedienen; und
einen austauschbaren Panelsatz, der konfiguriert ist, um manuell auf der Steuereinheit montiert und von ihr entfernt zu werden, aufweisend
ein Blutpanel (12);
ein Fluidpanel (20), das Blut- bzw. Fluidzufuhrschläuche aufweist, die daran befestigt sind und in Betriebseingriff mit der Blutpumpe (14) bzw. den Fluidpumpen (17, 18, 19) stehen;
ein Filterpatronenpanel (16), das an dem Blutpanel (12) und dem Fluidpanel (20) befestigt ist; und
eine austauschbare Filterpatrone (25), die darauf montiert ist,
**dadurch gekennzeichnet, dass**,
das interaktive Bediensystem Bedienereingaben zum Einstellen des Patientengewichts, der Blutpumpenströmungsrate und der Fluidpumpenströmungsraten beinhaltet; und die Steuerung konfiguriert ist, um die maximal zulässige Fluidzunahme oder den maximal zulässigen Fluidverlust, basierend ausschließlich auf Patientengewichtseinstellungen zwischen Null kg und etwa 20 kg, zu berechnen und anzuzeigen und einen Alarm- und/oder Stopp der Pumpen (14, 17, 18, 19) oder eine Beendigung der CRRT, als Reaktion auf die Patientenfluidzunahme oder den Patientenfluidverlust über die maximal zulässige Fluidzunahme oder den maximal zulässigen Fluidverlust hinaus, auszulösen.

2. Vorrichtung nach Anspruch 1, wobei die Steuerung für eine maximal zulässige Fluidzunahme oder einen maximal zulässigen Fluidverlust von etwa 7 ml/kg für Patientengewichtseinstellungen zwischen Null kg und etwa 20 kg sorgt.

3. Vorrichtung nach Anspruch 1, wobei die Steuerung für eine maximal zulässige Fluidzunahme oder einen maximal zulässigen Fluidverlust von etwa 42 ml für Patientengewichtseinstellungen zwischen Null kg und etwa 6 kg sorgt.

4. Vorrichtung nach Anspruch 3, wobei die Steuerung für eine maximal zulässige Fluidzunahme oder einen maximal zulässigen Fluidverlust von etwa 7 ml/kg für Patientengewichtseinstellungen zwischen etwa 6 kg und etwa 20 kg sorgt.

5. Vorrichtung nach Anspruch 1, wobei die Steuerung konfiguriert ist, um eine/einen erste/ersten und eine/einen zweite/zweiten Nettofluidzunahme/Nettofluidverlust einzustellen und ein Warnsignal auszulösen, wenn die Patientenfluidzunahme/ der Patientenfluidverlust größer als die erste Nettofluidzunahme/ der Nettofluidverlust ist, und einen Alarm auszulösen, wenn die Fluidzunahme/ der Fluidverlust größer als die zweite Nettofluidzunahme/ der zweite Nettofluidverlust ist.

6. Vorrichtung nach Anspruch 1, wobei die Fluidpumpen (17, 18, 19) eine Dialysierfluidpumpe, eine Ersatzfluidpumpe und eine Abflusspumpe aufweisen, wobei die Bedienereingaben eine Ersatzfluidrate, eine Dialysierrate und eine Nettofluidentfernungsrate aufweisen und wobei die Steuerung automatisch die Abflussentfernungsrate einstellt, ohne die vom Bediener eingestellte Dialysierfluidströmungsrate und die Ersatzfluidströmungsrate einzustellen, um die vom Bediener eingestellte Nettofluidentfernung basierend auf der folgenden Formel beizubehalten:
Nettoentfernung=Abfluss entfernt - Dialysat zugegeben -Ersatzfluid zugegeben.

7. Vorrichtung nach Anspruch 1, wobei die Steuerung konfiguriert ist, um eine Warnung als Reaktion auf Nettofluidzunahme oder einen Nettofluidverlust von mehr als etwa 20 ml auszulösen und den Alarm als Reaktion auf eine Nettofluidzunahme oder einen Nettofluidverlust von mehr als etwa 30 ml auszulösen bei Patientengewichtseinstellungen zwischen Null kg und etwa 10 kg.

8. Vorrichtung nach Anspruch 1, wobei die Steuerung konfiguriert ist, um eine Warnung als Reaktion auf eine Nettofluidzunahme oder einen Nettofluidverlust von mehr als etwa 35 ml auszulösen und den Alarm als Reaktion auf eine Nettofluidzunahme oder einen Nettofluidverlust von mehr als etwa 50 ml auszulösen bei Patientengewichtseinstellungen von zwischen etwa 10 kg und etwa 15 kg.

9. Vorrichtung nach Anspruch 1, wobei die Steuerung konfiguriert ist, um eine Warnung als Reaktion auf eine Nettofluidzunahme oder einen Nettofluidverlust von mehr als etwa 50 ml auszulösen und den Alarm als Reaktion auf eine Nettofluidzunahme oder einen Nettofluidverlust von mehr als etwa 80 ml auszulösen bei Patientengewichtseinstellungen von zwischen etwa 15 kg und etwa 20 kg.

10. Vorrichtung nach Anspruch 1, wobei die Steuerung für eine maximal zulässige Fluidzunahme oder einen maximal zulässigen Fluidverlust zwischen etwa 30 ml und etwa 40 ml bei Patientengewichtseinstellungen zwischen null kg und etwa 10 kg sorgt.

11. Vorrichtung nach Anspruch 1, wobei die Steuerung für eine maximal zulässige Fluidzunahme oder einen maximal zulässigen Fluidverlust zwischen etwa 50 ml und etwa 70 ml bei Patientengewichtseinstellungen zwischen etwa 10 kg und etwa 15 kg sorgt.

12. Vorrichtung nach Anspruch 1, wobei die Steuerung für eine maximal zulässige Fluidzunahme oder einen maximal zulässigen Fluidverlust zwischen etwa 80 ml und etwa 120 ml bei Patientengewichtseinstellungen zwischen etwa 15 kg und etwa 20 kg sorgt.

13. Vorrichtung nach Anspruch 1, wobei das interaktive Bedienersystem eine Bedienereingabe zum Einstellen einer Fluidzunahmegrenze oder Fluidverlustgrenze unterhalb des zulässigen Maximums beinhaltet.

14. Vorrichtung nach Anspruch 13, wobei die Steuerung konfiguriert ist, um den CRRT-Abschluss als Reaktion auf die eingestellte Fluidzunahme oder den eingestellten Fluidverlust, die/der über die Einstellung hinausgehen, während einer Zeitspanne von bis zu etwa 3 Stunden automatisch auszulösen.

15. Vorrichtung nach Anspruch 1, wobei die Steuerung konfiguriert ist, um eine Bedienereingabe zum Einstellen einer Fluidzunahme oder eines Fluidverlustes über das Maximum hinaus zu verhindern.

## Revendications

1. Appareil configuré pour exécuter des thérapies continues de remplacement rénal (CRRT) sur des patients pesant moins d'environ 20 kg, comprenant :
une unité de commande comprenant :
une pompe à sang (14) et une pluralité de pompes à fluide (17, 18, 19) montée sur celle-ci ;
un dispositif de commande configuré pour faire fonctionner ladite pompe à sang (14) et lesdites pompes à fluide (17, 18, 19) ; et
un système de commande interactif avec l'opérateur comprenant un écran d'interface d'opérateur connecté de manière fonctionnelle audit dispositif de commande, dans lequel ledit dispositif de commande comprend un logiciel configuré pour faire fonctionner ledit appareil en réponse à des entrées sélectionnées par l'opérateur ; et
un kit de panneau remplaçable configuré pour être manuellement monté sur et retiré de ladite unité de commande comprenant
un panneau pour le sang (12) ;
un panneau pour les fluides (20) comprenant une tubulure d'alimentation en sang et fluides, respectivement, fixée sur celui-ci et en prise fonctionnelle avec ladite pompe à sang (14) et lesdites pompes à fluide (17, 18, 19), respectivement ;
un panneau pour cartouche de filtre (16) fixé sur ledit panneau pour le sang (12) et ledit panneau pour les fluides (20) ; et
une cartouche de filtre remplaçable (25) montée sur celui-ci,
**caractérisé en ce que**,
ledit système de commande interactif avec l'opérateur inclut des entrées par l'opérateur pour régler le poids du patient, le débit de la pompe à sang et les débits des pompes à fluide ; et
ledit dispositif de commande est configuré pour calculer et afficher le gain ou la perte de fluide maximum admissibles uniquement sur la base de réglages de poids du patient entre zéro kg et environ 20 kg et déclencher une alarme et/ou une interruption desdites pompes (14, 17, 18, 19) ou un arrêt de la CRRT en réponse à un gain ou à une perte de fluide par le patient dépassant lesdits gain ou perte de fluide maximum admissibles.

2. Appareil selon la revendication 1 dans lequel ledit dispositif de commande fournit un gain ou une perte de fluide maximum admissibles d'environ 7 ml/kg pour des réglages de poids du patient entre zéro kg et environ 20 kg.

3. Appareil selon la revendication 1 dans lequel ledit dispositif de commande fournit un gain ou une perte de fluide maximum admissibles d'environ 42 ml pour des réglages de poids du patient entre zéro kg et environ 6 kg.

4. Appareil selon la revendication 3 dans lequel ledit dispositif de commande fournit un gain ou une perte de fluide maximum admissibles d'environ 7 ml/kg pour des réglages de poids du patient entre environ 6 kg et environ 20 kg.

5. Appareil selon la revendication 1 dans lequel le dispositif de commande est configuré pour régler un premier et un second gain net/une première et une seconde perte nette de fluide et déclencher un signal d'avertissement lorsqu'un gain ou une perte de fluide par le patient dépasse ledit premier gain net/ladite première perte nette de fluide et déclencher une alarme lorsqu'un gain ou une perte de fluide dépasse ledit second gain net/ladite seconde perte nette de fluide.

6. Appareil selon la revendication 1 dans lequel lesdites pompes à fluide (17, 18, 19) comprennent une pompe à fluide de dialysat, une pompe à fluide de remplacement et une pompe à effluent, dans lequel lesdites entrées par l'opérateur comprennent un taux de fluide de remplacement, un taux de dialysat et un taux net d'extraction de fluide et dans lequel ledit dispositif de commande ajuste automatiquement le taux d'extraction d'effluent sans ajuster le débit de dialysat réglé par l'opérateur et le débit de fluide de remplacement pour maintenir l'extraction nette de fluide réglée par l'opérateur sur la base de la formule :
extraction nette = effluent extrait - dialysat ajouté - fluide de remplacement ajouté.

7. Appareil selon la revendication 1 dans lequel ledit dispositif de commande est configuré pour déclencher un avertissement en réponse à un gain net ou à une perte nette de fluide dépassant environ 20 ml et déclencher ladite alarme en réponse à un gain net ou à une perte nette de fluide dépassant environ 30 ml à des réglages de poids du patient entre zéro kg et environ 10 kg.

8. Appareil selon la revendication 1 dans lequel ledit dispositif de commande est configuré pour déclencher un avertissement en réponse à un gain net ou à une perte nette de fluide dépassant environ 35 ml et déclencher ladite alarme en réponse à un gain net ou à une perte nette de fluide dépassant environ 50 ml à des réglages de poids du patient entre environ 10 kg et environ 15 kg.

9. Appareil selon la revendication 1 dans lequel ledit dispositif de commande est configuré pour déclencher un avertissement en réponse à un gain net ou à une perte nette de fluide dépassant environ 50 ml et déclencher ladite alarme en réponse à un gain net ou à une perte nette de fluide dépassant environ 80 ml à des réglages de poids du patient entre environ 15 kg et environ 20 kg.

10. Appareil selon la revendication 1 dans lequel le dispositif de commande fournit un gain ou une perte de fluide maximum admissibles entre environ 30 ml et environ 40 ml à des réglages de poids du patient entre zéro kg et environ 10 kg.

11. Appareil selon la revendication 1 dans lequel le dispositif de commande fournit un gain ou une perte de fluide maximum admissibles entre environ 50 ml et environ 70 ml à des réglages de poids du patient entre environ 10 kg et environ 15 kg.

12. Appareil selon la revendication 1 dans lequel le dispositif de commande fournit un gain ou une perte de fluide maximum admissibles entre environ 80 ml et environ 120 ml à des réglages de poids du patient entre environ 15 kg et environ 20 kg.

13. Appareil selon la revendication 1 dans lequel ledit système de commande interactif avec l'opérateur inclut des entrées par l'opérateur pour régler une limite de dépassement de gain ou de perte de fluide en dessous dudit maximum admissible.

14. Appareil selon la revendication 13 dans lequel ledit dispositif de commande est configuré pour déclencher automatiquement ledit arrêt de la CRRT en réponse à un réglage de gain ou de perte de fluide dépassant ledit réglage durant une période de temps allant jusqu'à environ 3 heures.

15. Appareil selon la revendication 1 dans lequel ledit dispositif de commande est configuré pour empêcher une entrée de l'opérateur pour régler un gain ou une perte de fluide dépassant ledit maximum.
